Europäisches Patentamt

European Patent Office

Office européen des brevets

(11) Veröffentlichungsnummer: **0 050 191**
**B1**

(12) **EUROPÄISCHE PATENTSCHRIFT**

(45) Veröffentlichungstag der Patentschrift:
24.10.84

(21) Anmeldenummer: 81105566.4

(22) Anmeldetag: 15.07.81

(51) Int. Cl.³: **A 61 K 9/28**, A 61 K 9/50,
A 61 K 31/60

(54) Verfahren zur Herstellung eines umhüllten Acetylsalicylsäurepräparates.

(30) Priorität: 16.10.80 DE 3039073

(43) Veröffentlichungstag der Anmeldung:
28.04.82 Patentblatt 82/17

(45) Bekanntmachung des Hinweises auf die Patenterteilung:
24.10.84 Patentblatt 84/43

(84) Benannte Vertragsstaaten:
AT CH DE FR GB LI SE

(56) Entgegenhaltungen:
DE - A - 2 044 707
FR - A - 2 145 642

CHEMICAL ABSTRACTS, Band 85, Nr. 12, 20. September 1976, Seiten 342, 343, Nr. 83190r Columbus, Ohio, U.S.A. J.P. DELPORTE et al.: "Effect of the formulation of enterosoluble preparations on the drug bioavailability. Part 4. Effect of the pH of dissolution of the applied enteric polymer on the in vivo absorption characteristics of acetylsalicylic acid tablets. Conclusions" CHEMICAL ABSTRACTS, Band 82, Nr. 10, 10. März 1975, Seite 469, Nr. 64453a Columbus, Ohio, U.S.A. K. LECHMANN et al.: "Use of aqueous dispersions of synthetic materials to coat drug forms" CHEMICAL ABSTRACTS, Band 85, Nr. 14, 4. Oktober 1976, Seite 317, Nr. 99090u Columbus, Ohio, U.S.A. E.

(73) Patentinhaber: Röhm GmbH, Kirschenallee
Postfach 4242, D-6100 Darmstadt 1 (DE)

(72) Erfinder: Dreher, Dieter, Waldstrasse 2 A,
D-6101 Bickenbach (DE)
Erfinder: Lehmann, Klaus, Dr., Schillerstrasse 85,
D-6101 Rossdorf (DE)
Erfinder: Bössler, Heide, Dr., Stefan-George-Weg 44,
D-6100 Darmstadt (DE)

(56) Entgegenhaltungen: (Fortsetzung)
KRISTOFFERSSON et al.: "On the effect of compression force and temperature on tablets with an acrylate plastic matrix. I. Tablets containing acetyl salicylic acid as model substance" CHEMICAL ABSTRACTS, Band 92, Nr. 6, 11. Februar 1980, Seite 334, Nr. 47217r Columbus, Ohio, U.S.A. J.P. DELPORTE und F. JAMINET, J. Phar. Belg. 1975 (30)2, S.99-113
J.P.DELPORTE und F.JAMINET, J.Pharm.Belg. 1976 (31)1, S.32-50
J.P.DELPORTE und F.JAMINET, J.Pharm.Belg. 1976 (31)3, S.263-276

## Beschreibung

Acetylsalicylsäurepräparate , am besten bekannt unter dem Handelsnamen «Aspirin», geschütztes Warenzeichen der Bayer AG, Leverkusen, gehören zu den weltweit gebräuchlichsten Arzneimitteln. Häufig besteht das Bedürfnis, Acetylsalicylsäurepräparate mit einem Überzug zu versehen, beispielsweise um den stark sauren Geschmack des Wirkstoffs zu überdecken oder um ein magensaftresistentes Präparat zu erzeugen. Acetylsalicylsäurepräparate mit Überzügen aus Celluloseacetat-phthalat, Hydroxypropylmethylcellulose-phthalat oder Methacrylsäure-Methacrylester-Copolymeren sind aus Arbeiten von J.P. Delporte und F. Jaminet in J. Pharm. Belg., 1975, Band 30,2, S. 99 bis 103; 1976, Band 31,1 und 3, S. 38 bis 50 und 263 bis 276, bekannt. Die Überzüge werden in allen Fällen aus Lösungen des Überzugsmittels in organischen Lösungsmitteln, wie Alkoholen und Estern, erzeugt. In neuerer Zeit wird jedoch angestrebt, die Verwendung von Arzneimittelüberzugslösungen in organischen Lösungsmitteln zu vermeiden.

Es sind auch Arzneimittelüberzugsmittel auf wässriger Basis bekannt, die das Überzugsmittel in dispergierter Form enthalten. Bei ihrer Anwendung kommt notgedrungen der zu überziehende Arzneimittelformling mit der wässrigen Phase in Berührung.

Die unmittelbare Verwendung derartiger Überzugsmitteldispersionen für Acetylsalicylsäure enthaltende Präparate musste dem Fachmann wegen der bekannten Hydrolyseempfindlichkeit der Acetylsalicylsäure ungeeignet erscheinen. Bekanntlich hydrolysiert Acetylsalicylsäure mit Wasser leicht zu Essigsäure und freier Salicylsäure, die eine schleimhautreizende Wirkung hat (vgl. «Hagers Handbuch der pharmazeutischen Praxis», Band II, 4. Neuausgabe, Springer-Verlag, 1969, S. 1017) und deshalb in Acetylsalicylsäurepräparaten nicht vorkommen soll. Zur Reinheitsprüfung von Acetylsalicylsäurepräparaten gehört stets die Prüfung auf freie Salicylsäure. Nach Böhme-Hartke (Kommentar zu EuAB, Band I und II, 1976, S. 479) soll der Grenzwert von 0,05% Salicylsäure in dem eingesetzten Wirkstoff nicht überschritten werden.

Acetylsalicylsäure hydrolysiert bereits an feuchter Luft (Böhme-Hartke, loc. cit.). Bei pH 2-3 ist sie am beständigsten; oberhalb pH 3 nimmt die Hydrolysegeschwindigkeit rasch zu und steigt oberhalb pH 9 sprunghaft an. Wegen der grossen Bedeutung der Acetylsalicylsäure-Hydrolyse ist ihr Verlauf und die Erfassung der Hydrolyseprodukte in der pharmazeutischen Literatur ausführlich behandelt (C.A. Kelly, Journal of Pharmaceutical Sciences, Band 59, 1970, S. 1053 bis 1079; R. Fernandez-Primi und E. Baumgartner, Journal Amer. Chem. Soc., Band 96, 1974, S. 4489 bis 4494). Alle Autoren kommen zu dem Schluss, dass ein lagerfähiges Acetylsalicylsäurepräparat auch keine Spuren von Wasser enthalten darf (Kelly, loc. cit., S. 1053, 1054) und dass Wasser bei der Zubereitung der Präparate vermieden werden muss (Böhme-Hartke, loc. cit., S. 480).

Das Vorurteil, das gegen die unmittelbare Umhüllung von Acetylsalicylsäurepräparaten mit wässrigen Überzugsmitteldispersionen bestand, lässt sich aus der DE-PS 2 135 073 ablesen, wo in Spalte 7 empfohlen wird, auf wasserempfindliche Wirkstoffe bzw. Arzneiformen vor der Umhüllung mit wässrigen Kunststoffdispersionen einen Schutzfilm aus einem Polymeren, das in einem organischen Lösungsmittel gelöst ist, aufzubringen. Je grösser das Verhältnis von Oberfläche zu Volumen des zu überziehenden Präparats ist, also je feinteiliger es ist, um so grösser sollte die Hydrolysegefahr sein. Deshalb gilt als Vorurteil für Kristalle und Granulate con Acetylsalicylsäure in noch höherem Masse als für gepresste Drageekerne oder Tabletten. Obwohl wässrige Überzugsmitteldispersionen für Arzneimittel heute in grossem Umfang angewendet werden, sind damit umhüllte Acetylsalicylsäurepräparate oder Versuche zu ihrer Herstellung bisher nicht bekannt geworden.

Es wurde nun gefunden, dass sich umhüllte Acetylsalicylsäurepräparate durch unmittelbares Aufbringen einer wässrigen Dispersion eines Arzneimittelüberzugsmittels auf nicht überzogene Arzneiformen mit Acetylsalicylsäure als Wirkstoff und Verdampfen des in der Dispersion enthaltenen Wassers ohne Bildung eines Überzugsmittelfilms herstellen lassen, ohne dass dabei freie Salicylsäure im stärkeren Masse als bei Verwendung üblicher organischer Überzugsmittellösungen gebildet wird. Die üblichen Auftragsverfahren, beispielsweise im Dragierkessel oder das Wirbelschichtverfahren, können angewendet werden. Dieser Befund ist überraschend und war angesichts der erwiesenen Hydrolyseempfindlichkeit der Acetylsalicylsäure nicht zu erwarten.

Die zu überziehenden Arzneiformen enthalten in der Regel Acetylsalicylsäure als einzigen Wirkstoff. Der Gehalt an freier Salicylsäure im fertigen Präparat soll unter 0,2 Gew.-%, bezogen auf Acetylsalicylsäure, liegen; in den überzogenen Arzneiformen beträgt er im allgemeinen 0,01 bis 0,05%. Daneben können weitere Wirkstoffe enthalten sein. Die Erfindung bezieht sich auf das Beschichten von nicht überzogenen Arzneiformen, d.h. solchen, die keinen geschlossenen Filmüberzug tragen und in denen Acetylsalicylsäurekristalle entweder an der Oberfläche liegen oder in einer porösen Matrix durch Poren zugänglich sind.

Im einfachsten Fall sind es Acetylsalicylsäurekristalle von ausreichender Grösse. Sie sollten zu wenigstens 90% grösser als 0,1 mm und bevorzugt grösser als 0,3 mm sein. Auch Formlinge in einer Grösse bis zu etwa 3 mm Korndurchmesser, wie z.B. Sieb-, Teller- oder Lochscheibengranulate oder Pellets sind besonders geeignete Ausgangsformen für die erfindungsgemässen Präparate. Tabletten können ebenfalls im Sinne der Erfindung überzogen werden. Die erwähnten Formlinge können übliche Hilfsstoffe, gegebenenfalls sogar in überwiegender Menge enthalten. Als Beispiele seien Bindemittel, Füllstoffe, Sprengmittel, Gleitmittel und Zuckerkristalle genannt.

Die Acetylsalicylsäure enthaltenden Arzneiformen werden mittels bekannter Verfahren mit der wässrigen Überzugsmitteldispersion überzogen, wobei für eine möglichst schnelle Trocknung Sorge getragen werden soll. Das Wirbelschichtverfahren ist — insbesondere bei der Anwendung auf Arzneiformen bis 3 mm Korndurchmesser — bevorzugt, jedoch lässt sich auch im rotierenden Kessel ein guter Überzug erzeugen, wobei die Dispersion vorzugsweise aufgesprüht wird. Am günstigsten ist eine Sprühgeschwindigkeit von 1 bis 3 g/min und kg der Arzneiformen bei einer Zuluftmenge von 0,1 bis 1 m³/min und kg. Die zum Trocknen eingesetzte Luft wird vorzugsweise leicht erwärmt, und zwar beim Wirbelschichtverfahren auf etwa 30 bis 50°C, beim Kesselverfahren auf 40 bis 70°C. Das überzogene Gut erreicht dabei Temperaturen von 25 bis 30 bzw. 30 bis 40°C.

Die Menge des aufgetragenen Überzugsmittels richtet sich nach dem damit verfolgten Zweck. Für Isolierüberzüge, beispielsweise auf Pellets oder Tabletten, reichen Mengen ab 0,1 mg/cm², berechnet als reines Überzugsmittel ohne Hilfsstoffe, aus. Darüber hinaus können weitere Überzüge aus anderen Substanzen aufgebracht werden. Vorzugsweise werden magensaftresistente Überzüge aufgebracht, die 1 bis 20% und insbesondere 2 bis 5% des Gewichts des nicht überzogenen Ausgangsteilchens ausmachen. Bei grösseren Pellets oder Tabletten werden 1 bis 20 mg/cm², vorzugsweise 2 bis 6 mg/cm² für diesen Zweck benötigt. Diese Werte gelten auch für permeable, retardierende Filmüberzüge. In allen Fällen beziehen sich diese Angaben auf das Trockengewicht des reinen filmbildenden Überzugsmittels. Die aufgetragene Dispersion kann darüber hinaus weitere Hilfsstoffe enthalten, deren Menge das Gewicht des Überzugsmittels übertreffen kann. Solche Hilfsstoffe sind Emulgatoren, Weichmacher, Filmbildungshilfsmittel, Pigmente, Füllstoffe, Gleitmittel oder Trennmittel.

Der Gehalt an dispergiertem Filmbildner in der wässrigen Überzugsmitteldispersion liegt im allgemeinen zwischen 5 und 30 Gew.-%, vorzugsweise zwischen 10 und 20 Gew.-%, während der Gesamtfeststoffgehalt einschliesslich aller Hilfsstoffe im Bereich von 15 bis 40 Gew.-% liegt. Zur Stabilisierung enthalten die Dispersionen in der Regel wasserlösliche, toxikologisch unbedenkliche Emulgiermittel, wie Natriumlaurylsulfat, Natriumstearylsulfat, Natrium-cetylstearylsulfat, hochoxäthylierte Alkyl-phenole, oxäthylierte Fettsäuren oder Polyoxäthylensorbitan-monostearat oder -monooleat. Der Filmbildner kann auch durch eingebaute polare Gruppen, insbesondere Sulfonsäuregruppen, selbstemulgierend sein, so dass niedermolekulare Emulgiermittel entbehrlich sind. Da die Hydrolyseempfindlichkeit der Acetylsalicylsäure mit steigendem pH-Wert zunimmt, sind Dispersionen mit einem pH-Wert unter 7 und insbesondere unter 4 besonders bevorzugt. Die geringste Hydrolysegefahr besteht in dem Stabilitätsmaximum zwischen pH 2 und 3.

Das dispergierte Überzugsmittel soll wenigstens oberhalb 40°C und vorzugsweise schon bei 20°C filmbildend sein. Beim pH-Wert der wässrigen Phase muss es in Wasser unlöslich bis höchstens quellbar sein. Bevorzugt sind magensaftresistente und darmsaftlösliche Überzugsmittel. Das sind insbesondere solche, die, bezogen auf das Gewicht des Polymerisats, 8 bis 30 Gew.-%, vorzugsweise wenigstens 10 Gew.-% Carboxylgruppen enthalten. Gute magensaftresistente Überzugsmittel enthalten 10 bis 25 Gew.-% Carboxylgruppen, retardierende Überzüge weniger als 20 Gew.-% Carboxylgruppen. Eine ausführliche Beschreibung geeigneter Überzugsmitteldispersionen und ihrer Herstellung findet sich in der DE-PS 2 135 073.

Zu den geeigneten Überzugsmitteln gehören z.B. Schellack, Copolymerisate von Methylvinyläther und Maleinsäurehalbestern, von Polyvinylacetal und α-(Dialkylamino)-essigsäure-vinylestern, von Äthylen und Maleinsäure oder von Vinylacetat und Vinylpyrrolidon. Bevorzugte Überzugsmittel sind solche auf Basis von Celluloseestern, beispielsweise Cellulose-acetatphthalat, und von Polymerisaten bzw. Copolymerisaten von Acryl- und Methacrylmonomeren, wie Acryl- und Methacrylsäure und ihren niederen Alkylestern.

Die aus Granulaten oder Kristallen von Acetylsalicylsäure hergestellten Präparate weisen unmittelbar nach der Herstellung einen meistens unveränderten und manchmal geringfügig bis auf 0,05% angestiegenen Gehalt an freier Salicylsäure auf. Der Wassergehalt beträgt im allgemeinen nicht mehr als 0,5 Gew.-%. Nach einjähriger Lagerzeit an trockener Luft wurden Salicylsäuregehalte unter 0,05% und nach dreijähriger Lagerung unter 0,1% gefunden. Das entspricht den Werten, die man auch beim Überziehen der gleichen Ausgangspräparate mit organischen, wasserfreien Überzugsmittellösungen nach gleichlangen Lagerzeiten findet.

Die überzogenen Arzneiformen können als solche verwendet werden. Wenn die einzelne Arzneiform weniger als die erwünschte Dosismenge enthält, z.B. im Falle überzogener Kristalle, Granulate oder Pellets, werden diese zweckmässigerweise zu grösseren Dosierungseinheiten zusammengefasst. Dazu eignet sich das Abfüllen in Kapseln oder das Verpressen zu Tabletten. Im allgemeinen enthält eine Dosierungseinheit wenigstens 200 mg Acetylsalicylsäure. Eine bevorzugte Dosierungsgrösse beträgt etwa 500 mg Acetylsalicylsäure.

*Beispiele*

1. *Überziehen von ASS-Granulat im Wirbelschichtverfahren*

Auf ein Acetylsalicylsäure (ASS)-Granulat mit einer Korngrösse von 0,5-1,2 mm und einem Gehalt an freier Salicylsäure (SS) von 0,02 Gew.-% wurden in einer Wirbelschichtapparatur 10 Gew.-%, in einem weiteren Ansatz 20 Gew.-% (bezogen auf das eingesetzte Granulat) einer 30%igen wässrigen Dispersion eines Mischpolymerisats aus
45 Gew.-% Methacrylat und
55 Gew.-% Acrylsäureäthylester,
die als Emulgatoren (bezogen auf Lacksubstanz) 0,5 Gew.-% Natriumlaurylsulfat und 2,5 Gew.-% Polyäthylen-sorbitan-monostearat enthielt und einen pH-Wert von 2,5 hatte, aufgesprüht. Die Zulufttemperatur betrug 40°C, die Ablufttemperatur 28°C. Das fertige Präparat enthielt 0,4 Gew.-% Wasser. Nach einer Lagerzeit von 50 Tagen in geschlossenem Behälter bei Raumtemperatur wurde an beiden Präparaten ein SS-Gehalt von 0,01 Gew.-% und ein unveränderter Wassergehalt von 0,4 Gew.-% gefunden.

## 2. Überziehen von ASS-Kristallen im Wirbelschichtverfahren

ASS-Kristalle einer Korngrösse von 0,3-0,8 mm mit einem SS-Gehalt von 0,01% wurden in einer Wirbelschichtapparatur mit 2,5 Gew.-% einer 30%igen wässrigen Dispersion eines Mischpolymerisats aus

62 Gew.-% Acrylsäureäthylester

37 Gew.-% Methacrylsäuremethylester

1 Gew.-% Methacrylsäure

die (bezogenn auf Lacksubstanz) 0,4 Gew.-% Natriumlaurylsulfat und 6 Gew.-% Polyoxyäthylen-sorbitan-monooleat als Emulgatoren enthielt und einen pH-Wert von 2,5 hatte, überzogen. Die Zulufttemperatur betrug 40°C, die Ablufttemperatur 30°C.

Der SS-Gehalt in den überzogenen Kristallen betrug 0,04%, der Wassergehalt 0,21 Gew.-%. Wurde eine gleichartige Dispersion, die mit NaOH auf pH 7,4 eingestellt war, als Überzugsmittel verwendet, so wurde in dem überzogenen Material ein nahezu gleichgrosser Gehalt von 0,3% SS gefunden.

## 3. Überziehen von ASS-Kristallen im Dragierkessel

Eine Charge von 3 kg ASS-Kristallen (Wassergehalt 0,35%, SS-Gehalt 0,005%, Korngrösse 0,3 bis 0,8 mm) wurde in einem Labor-Dragierkessel mit 35 cm Durchmesser bei 38 Upm mit einer wässrigen Suspension aus

250 g der in Beispiel 1 verwendeten Dispersion

75 g einer 10%igen wässrigen Lösung eines Polyäthylenoxids vom Molekulargewicht 6000

30 g Talkum

1,5 g Silicon-Antischaumemulsion

243,5 g Wasser

besprüht. Der Gesamtfeststoffgehalt der Dispersion betrug 19 Gew.-%. Während des Einsprühens, das 143 min dauerte, wurde Warmluft von 50°C eingeblasen. Anschliessend wurden die beschichteten Kristalle noch 6 Stunden im Umlufttrockenschrank bei Raumtemperatur getrocknet.

Die aufgetragene Menge des Filmbildners lag bei 2,5 Gew.-%, die Gesamtmenge des Überzugs bei 3,8 Gew.-% Trockensubstanz. Der Wassergehalt des fertigen Präparats betrug 0,3%, der SS-Gehalt 0,01 Gew.-%.

## 4. Überziehen von ASS-Tabletten im Dragierkessel

Eine Charge von 3 kg ASS-Tabletten mit einem Wirkstoffgehalt von 500 mg ASS pro Tablette (Tablettengewicht: 620 mg) und einem Salicylsäuregehalt von 0,025% wurden in einem Labor-Dragierkessel mit 35 cm Durchmesser bei 35 Upm mit einer wässrigen Suspension aus

335 g der im Beispiel 1 verwendeten Dispersion

33 g einer 33%igen wässrigen Lösung eines Polyäthylenoxids vom Molekulargewicht 6000

100 g Talkum

292 g Wasser

besprüht. Der Gesamtfeststoffgehalt der Suspension betrug 28%. An Lacktrockensubstanz wurden 5 mg/cm² Tablettenoberfläche, entsprechend 3,35 Gew.-%, aufgetragen. Die Gesamtmenge des Überzugs lag bei 7,0% Trockensubstanz. Der Salicylsäuregehalt des fertigen Präparats betrug 0,03% und war nach elfwöchiger Lagerung bei Raumtemperatur unverändert. Nach 122 Wochen Lagerung bei Raumtemperatur wurden 0,05% SS gefunden.

## Patentansprüche

1. Verfahren zur Herstellung eines umhüllten Acetylsalicylsäurepräparats, gekennzeichnet durch Aufbringen eines filmbildenden Arzneimittelüberzugsmittels in Form einer wässrigen Dispersion auf nicht überzogene Arzneiformen, die Acetylsalicylsäure als Wirkstoff enthalten, und Verdampfen des in der Dispersion enthaltenen Wassers unter Bildung eines Überzugsmittelfilms und gegebenenfalls Vereinigen der überzogenen Arzneiformen zu grösseren Dosiereinheiten.

2. Verfahren nach Anspruch 1, dadurch gekennzeichnet, dass die wässrige Dispersion einen pH-Wert unter 7 hat.

3. Verfahren nach den Ansprüchen 1 oder 2, dadurch gekennzeichnet, dass die wässrige Dispersion im Wirbelschichtverfahren aufgebracht wird.

4. Verfahren nach den Ansprüchen 1 bis 3, dadurch gekennzeichnet, dass Arzneiformen mit einem Durchmesser unter 3 mm überzogen werden.

5. Verfahren nach den Ansprüchen 1 bis 4, dadurch gekennzeichnet, dass das dispergierte Überzugsmittel wenigstens 0,5 Gew.-%, vorzugsweise 8 bis 30 Gew.-%, bezogen auf das Trockengewicht, an Carboxylgruppen enthält.

6. Verfahren nach den Ansprüchen 1 bis 5, dadurch gekennzeichnet, dass das dispergierte filmbildende Überzugsmittel ein Polymerisat bzw. Copolymerisat von Acryl- und/oder Methacrylmonomeren ist.

7. Verfahren nach den Ansprüchen 5 und 6, dadurch gekennzeichnet, dass ein dispergiertes magensaftresistentes, darmsaftlösliches Überzugsmittel eingesetzt wird.

8. Verfahren nach den Ansprüchen 5 und 6, dadurch gekennzeichnet, dass eine Überzugsmitteldispersion eingesetzt wird, die einen permeablen Überzug ergibt, der eine Geschmacksabdeckung und/oder retardierte Freisetzung des Wirkstoffs im Verdauungstrakt bewirkt.

## Claims

1. Process for the preparation of a coated acetylsalicylic acid preparation, characterised by the application of a film-forming pharmaceutical coating agent in the form of an aqueous dispersion to non-coated pharmaceutical preparations which contain acetylsalicylic acid as active substance, and evaporation of the water contained in the dispersion to form a coating film and possibly combining the coated pharmaceutical preparations to form larger dosage units.

2. Process as claimed in claim 1, characterised in that the aqueous dispersion has a pH of less than 7.

3. Process as claimed in claim 1 or 2, characterised in that the aqueous dispersion is applied by the fluidised bed method.

4. Process as claimed in claims 1 to 3, characterised in that pharmaceutical preparations with a diameter of less than 3 mm are coated.

5. Process as claimed in claims 1 to 4, characterised in that the dispersed film-forming coating agent contains at least 0.5% by weight, preferably 8 to 30% by weight, based on the dry weight, of carboxyl groups.

6. Process as claimed in claims 1 to 5, characterised in that the dispersed film-forming coating agent is a polymer or copolymer of acrylic and/or methacrylic monomers.

7. Process as claimed in claims 5 and 6, characterised in that a dispersed coating agent which is resistant to gastric juices and soluble in intestinal juices is used.

8. Process as claimed in claims 5 and 6, characterised in that a dispersion of coating agent is used which yields a permeable coating which masks the flavour of the active substance and/or brings about the delayed release of the active substance in the digestive tract.

## Revendications

1. Procédé pour l'obtention d'une préparation enrobée d'acide acétylsalicylique, caractérisé par l'application d'une substance filmogène de revêtement pour médicaments, sous la forme d'une dispersion aqueuse, sur des formes médicamenteuses non revêtues qui contiennent de l'acide acétylsalicylique en tant que substance active, évaporation de l'eau contenue dans la dispersion avec formation d'une pellicule de la substance de revêtement et, le cas échéant, réunion des formes médicamenteuses revêtues en unités de dosage plus grandes.

2. Procédé selon la revendication 1, caractérisé en ce que la dispersion aqueuse a un pH inférieur à 7.

3. Procédé selon la revendication 1 ou 2, caractérisé en ce que la dispersion aqueuse est appliquée par le procédé en couche fluidisée.

4. Procédé selon l'une quelconque des revendications 1 à 3, caractérisé en ce qu'on revêt des formes médicamenteuses ayant un diamètre inférieur à 3 mm.

5. Procédé selon l'une quelconque des revendications 1 à 4, caractérisé en ce que la substance filmogène de revêtement dispersée contient au moins 0,5% en poids et, de préférence, 8 à 30% en poids, par rapport au poids de substance sèche, de groupes carboxyles.

6. Procédé selon l'une quelconque des revendications 1 à 5, caractérisé en ce que la substance filmogène de revêtement dispersée est un polymère ou copolymère de monomères acryliques et/ou méthacryliques.

7. Procédé selon la revendication 5 ou 6, caractérisé en ce qu'on utilise en dispersion une substance de revêtement qui est résistante au suc gastrique et soluble dans le suc intestinal.

8. Procédé selon la revendication 5 ou 6, caractérisé en ce qu'on utilise une dispersion de substance de revêtement qui donne un revêtement perméable assurant un masquage du goût et/ou une libération retardée de la substance active dans le tube digestif.